# EUROPEAN PATENT APPLICATION

(11) **EP 2 347 664 A1**
(43) Date of publication of application: **27.07.2011**
(21) Application number: 09823650.8
(22) Date of filing: 29.10.2009
(51) Int. Cl.: A41B 9/06, A41B 9/02, A41D 7/00, A41D 13/00, A41D 13/02

(54) **GARMENT**

(30) Priority: 30.10.2008 JP 2008279377
(71) Applicant: Toray Industries, Inc., Tokyo, 103-8666 (JP)
(72) Inventor: SUZUKI, Hidetoshi, Tokyo 103-8666 (JP); KOYAMA, Yoshiro, Kyoto-shi Kyoto 604-8175 (JP); TANAKA, Masami, Kyoto-shi Kyoto 604-8175 (JP); KOUNO, Chisei, Tokyo 151-0063 (JP); ATOMI, Yoriko, Tokyo 151-0053 (JP); SAKURAI, Takashi, Tokyo 116-0003 (JP)
(74) Representative: Webster, Jeremy Mark
(86) International application number: PCT/JP2009/068551
(87) International publication number: WO 2010/050540

(57) **Abstract**

A garment provided with a main part which consists of a front body section, a back body section and sleeves extending from front and back body sections toward both arm sides over shoulders, and a pair of first tightening parts separately placed in right and left areas located at the positions of right and left deltoid muscles covering the boundary of the wearer's shoulder and upper arm in the back side, wherein the front and back body sections and the upper arm sections of the sleeves comprise at least two kinds of stretch materials, which differ in stretchability from each other, including a material for the main part and a material for the first tightening parts having a lower stretchability than the main part. Thus, it is possible to provide a daily wearable garment which can inductively elevate the wearer's chest without giving a tight feeling to thereby improve body function.

## Description

### TECHNICAL FIELD

The present invention relates to garments, in particular, to a garment capable of holding a posture in which the chest is elevated and the back is straightened and capable of improving a body function by simply being worn, and particularly suitably used as an undershirt.

### BACKGROUND ART

Various types of inner wears for correcting a posture or for supporting and assisting the movement of the muscles during exercise and during walking are conventionally provided.

In the posture correcting garment of Japanese Patent Publication No. 2006-320640 (patent document 1), a tension section having a shape of a laterally facing alphabet H, including tension parts 100, 102 separately located in left and right horizontal directions at an upper part and a lower part on a neck side and a tension part 102 for coupling middles of the upper and lower tension parts 100, 101 is attached to an inner surface of a main body 105. It describes that left and right scapulae of the wearer are surrounded with the tension parts 101 to 102, so that the left and right scapulae are pulled closer toward the inner side in the right and left directions at a rear surface side and a movable range of the shoulder joint is widened thereby improving a standing posture and supporting the erector muscle of the spine.

Furthermore, the movable region of the arm joint is widened by not arranging the tension section at the position covering an armhole so as not to inhibit the movement of deltoid muscles.

The undershirt disclosed in Japanese Laid-Open Patent Publication No. 2004-44070 (patent document 2) has a function of actively improving the movement of the shoulders and the arms at the time of pitching and at the time of impact. Such undershirt is made from three types of, first to third, stretch materials in which the tightening force is differed in three stages, where the tightening force is first material < second material < third material.

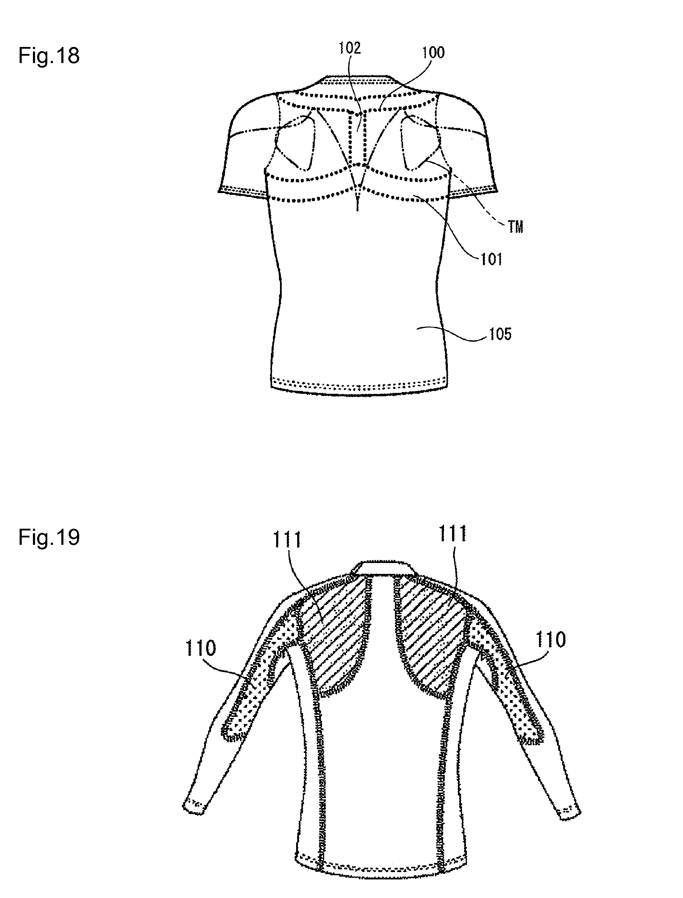

As shown in Fig. 19, the second material is the material of the main part, where a region 110 extending from a portion of triceps brachii muscle towards an elbow is formed by the first material having a weaker tightening force than the material of the main part, and a pair of left and right regions 111 of large area extending from the inner side of the deltoid muscles toward the scapula in continuation to the region 110 on the arm side is formed by the third material with stronger tightening force.

The garment provided with a high tightening force part is also proposed in Japanese Laid-Open Patent Publication No. 9-25008 (patent document 3), Japanese Laid-Open Patent Publication No. 2004-263362 (patent document 4), and the like, but the configuration is such that the band-shaped high tightening force part is traversed across the rear surface from left to right and extended to the arm.

### Patent document

Patent document 1: Japanese Laid-Open Patent Publication No. 2006-320640
Patent document 2: Japanese Laid-Open Patent Publication No. 2004-44070
Patent document 3: Japanese Laid-Open Patent Publication No. 09-25008
Patent document 4: Japanese Laid-Open Patent Publication No. 2004-263362

### DISCLOSURE OF THE INVENTION

### PROBLEMS TO BE SOLVED BY THE INVENTION

However, if the tension section including the tension parts 101 to 102 is arranged over the entire region of the upper side region of the back as in the posture correcting garment of patent document 1, the tightening feeling of the entire upper side region of the back becomes strong. If the tightening feeling becomes strong, the wearer tends to hunch to take a posture that alleviates the tightening feeling, in which case, the tightening feeling may increase, as opposed to a posture of elevating the chest and widening the left and right scapulae.

Furthermore, the tension section does not cover from the shoulder to the sleeve so as not to inhibit the movement of the deltoid muscles to widen the movable range of the shoulder joint, but if the arm drops forward at the time of fatigue and the like, the deltoid muscles may adduct and the left and right scapulae may be pulled by the deltoid muscles so as to easily be in a left and right widening direction as the tension section is not provided between the shoulder joints. In such a case, it is difficult to expand the left and right scapulaes to improve a posture unless the force of pulling the left and right scapulae to each other is made stronger by the tension section. If the force of the tension section is made stronger to improve a posture, however, the tightening feeling at the upper region of the back may further increase.

The undershirt of patent document 2 has a function of actively assisting the movement of the shoulders and the arms at the time of hitting and at the time of impact, but the tightening feeling on the back side is strong, similar to patent document 1, as the region 11 of large area having strong tightening force is spread over the region excluding the central back muscle portion of the upper region of the back, and hence it cannot be worn daily. Furthermore, since the region 111 of large area that is in contact with the scapulae is arranged on both left and right sides with the central part of the back having weak tightening force in between, the wearer feels as if a strong force is loaded on both left and right sides of the back, and the left and right scapulae tend to easily adduct with the central part of weak tightening force in between.

If a high tightening part is arranged traversing the back as in the garments of patent document 3 and patent document 4, the tightening feeling on the back side becomes stronger and the comfortableness as an underwear to wear daily is damaged, similar to patent documents 1, 2.

Therefore, the garment having the functionality to assist exercise or to correct the posture provided in the related art has a portion with increased tightening force to be arranged on a back surface side arranged in large area at the upper side region of the back, or is arranged so as to traverse the back by taping, and hence the tightening feeling and the oppressing feeling on the back side are strong, and an uncomfortable feeling is occurs when worn daily for a long period of time. Thus, improvement can be made as a garment to be worn daily in order to improve the body function and not limited to during exercise.

It is an object of the present invention to provide a garment that can be worn daily without the tightening feeling and with satisfactory fitting, the garment being capable of improving the body function at the time of daily performance such as a sitting posture, a standing posture, and a walking posture when worn.

Specifically, it is an object to provide a garment capable of increasing an adduction of the scapulae and maintaining the backward movement of the shoulders by wearing, and inducing the elevation of the rib cage and obtaining a posture in which the back is straightened, and improving the body function by reducing heart rates and breath rate based thereon.

### MEANS FOR SOLVING THE PROBLEMS

In order to solve the above problems, a first invention provides a garment including,
a main part which includes front and back body sections, and sleeves extending from the front and back body sections towards both arm sides over shoulders; and
a pair of first tightening parts separately placed in left and right areas located at the positions of right and left deltoid muscles covering a boundary of the wearer's shoulder and upper arm in the back side, wherein
the front and back body sections and the upper arm sections of the sleeves include at least two kinds of stretch materials, which differ in stretchability from each other, including a material for the main part and a material for the first tightening parts having a lower stretchability than the main part. Accordingly, the first tightening parts are more strongly contact with the deltoid muscles than other sites.

The front and back body sections and the sleeves may be separate bodies or may integrally configure the main part.

As mentioned above, the garment of the present invention is formed from the main part other than the left and right shoulders and the areas of the deltoid muscles of the upper arm, and a high tightening part of large area or a high tightening part that traverses the back are not provided at the back. The tightening feeling and the oppressing feeling on the back side are thus removed. If the main part is formed from a material having satisfactory stretchability and preferably a thin material, the garment can be fitted to the body of the wearer so that the wearer does not feel an uncomfortable feeling, and hence can be worn daily.

Tightening parts with lower stretchability than other sites are arranged at the area corresponding to the deltoid muscles at the back of the wearer so as to strongly be contact with and stimulate the deltoid muscles at the back of the wearer than other sites. The wearer then can constantly be conscious of the deltoid muscles at the back, so that the left and right scapulae can be induced in the adducting direction of approaching each other and the left and right shoulders can be induced to spread towards the back side.

The first tightening part may appropriately disperse the tightening force by being formed to a point group.

The deltoid muscles at the back are the sites bridging between the shoulder regions and the sleeve regions with the boundary position (shoulder) of the front and back body sections and the sleeves in between on the garment side.

Normally, a posture tends to be a slouching posture leaning towards the front side at the time of fatigue or stress, and the left and right scapulae tend to bend towards the front side and the rib cage tends to easily narrow. In such a case, the wearer can be conscious so as not to take the slouching posture of leaning forward by constantly stimulating the deltoid muscles at the back.

Furthermore, since the first tightening parts of low stretchability are arranged at the site of the deltoid muscles continuing to the left and right scapulae, even when the left and right scapulae attempt to bend toward the front side, the first tightening parts do not follow the movement of the left and right scapulae, and induce the left and right scapulae in the adducting direction of approaching each other, and pull the left and right scapulae toward the back side thereby aiding in the direction of elevating the rib cage.

Moreover, in the daily movement such as a sitting posture, a standing posture, and a walking other than at the time of fatigue and stress, the first tightening parts induce the left and right scapulae toward the back side thereby elevating the rib cage, so that a posture in which the back muscle is straightened can be constantly held.

With a posture in which the rib cage is elevated and the back muscle is straightened, the heart rates can be prevented from increasing and can be further reduced during sitting, standing, and walking.

Similarly, with regards to a breath rate, the breath rate can be prevented from increasing and can be further reduced during the daily behavior.

With regards to the heart rate and the breath rate, the breathing tends to become light or smothering may occur and the heart rate and the breath rate increase in the stress state. The heart rate and the breath rate gradually increase when standing up suddenly or even when walking, whereby breathing becomes heavy and walking may not be continued.

In such a case, as mentioned above, an increase in the heart rate and breath rate can be suppressed and further reduced, and the body function can be enhanced by wearing the garment of the present invention.

Therefore, the tense feeing can be alleviated and a relaxed stable state can be obtained so as to be in a physically and mentally cleared state by wearing the garment of the present invention. As a result, the work can be continued positively and efficiently when worn during work.

Furthermore, as walking is recommended to enhance health in recent years, long time duration of walking and exercise can be carried out without effort. It is actually found that the step and the walking speed also increase.

When in the state where the rib cage is elevated and the chest is elevated, the upper arms from the left and right shoulders to the elbows are also induced to the body side position from the front side.

Therefore, a posture in which the chest is elevated, the back muscle is straightened and the left and right hands are at the body side positions becomes a correct posture in appearance during sitting and during standing, and gives a favorable impression.

During walking, the left and right shoulders are induced to the back side, so that the left and right hands can also be greatly swung to the back side thus giving a favorable impression of walking lively.

The first tightening part with lower stretchability than the main part is preferably formed from one of the means selected from (1) to (4).
(1) A patch made from the same material as or a different material from the main part is overlapped on an outer surface or an inner surface of the main part, and then sewed, or adhered or thermal compression bonded using a resin adhesive.
(2) A patch made from a different material from the main part is filled and arranged in an opening provided in the main part, and joining end edges are sewed.
(3) A fabric is changed from the main part with the same material as or a different material from the main part.
(4) An elastic resin is applied to the main body.

A synthetic fiber such as polyester and polyamide, a cellulose fiber such as rayon and cotton, and a natural fiber such as wool and silk are appropriately used for the main material in the material of the main part with satisfactory stretchability. These materials may be mixed by combined knitting, insertion, pulling and the like for use. The stretch material is preferably mixed to give stretchability to the material of the main part. A polyurethane elastic fiber, a polyester elastomeric fiber, a PTT complex finished yarn (side by side bimetal yarn having poly trimethylene terephthalate as main component) and the like are appropriately used for the stretch material.

If the material of the main part is made thin and lighter weight, the wear comfort can be improved without applying a load on the wearer. Specifically, fabric weight is preferably 100 to 200 g/m². This is because the main part likely becomes sheer and tends to easily rip if smaller than 100 g/m², and becomes thick and heavy and thus causing a massive feeling and hardness and affecting the comfortability if greater than 200 g/m². Fabric weight of the main part is more specifically 120 to 170 g/m².

The first tightening part is made to have lower stretchability than the main part using the methods (1) to (4).

When overlapping with the main part using the patch of (1), the tightening force can be enhanced even if the patch is the same material as the main part, and the tightening force become stronger if the patch is overlapped on the main part using the material with lower stretchability than the main part.

When adhering the patch to the main part using the resin adhesive, a thermoplastic hot melt resin is suitably used for the resin adhesive, and may be a hot melt resin of polyurethane, polyester, polyolefin, styrene and the like.

The method of applying the resin adhesive prevents hardness of the fabric of the main part, where the resin is preferably applied in dot-form rather than being applied over the entire surface to ensure air permeability of the laminated portion with the fabric.

When filling and arranging the patch formed with a different material at the opening provided in the main part and sewing the joining edges of (2), the tightening force becomes stronger by using the material of lower stretchability than the main part for the patch.

When changing the fabric from the main part with the same material as or a different material from the main part of (3), the stretchability of the portion where the fabric is changed is made lower than the main part to provide high tightening force.

In the means for changing the fabric, the tightening part preferably adopts the fabric of reducing the loop length or increasing the number of sinker loops per unit area to suppress deformation of the loop.

The knitting yarn may be changed for the main part and the tightening part, where the knitting yarn of low stretchability may be used for the tightening part or a thermal fusion yarn may be used and heated after knitting to be integrally cured.

Changing the fabric or the knitting yarn so that the first tightening part is in point group is more preferable since the tightening part can be appropriately dispersed.

When applying the elastic resin to the main part of (4) to give high tightening force, the elastic resin for use includes silicone, urethane, acryl, nylon, and the like. A cross linking agent or a penetrating agent may be contained in the elastic resin as necessary to enhance the permeability and the application intensity to the fabric of the main part.

Spraying, transferring, screen printing, and rotary printing may be appropriately used for the application method.

Patterns in a mode (area application) of applying all over the range to exert the tightening force, a mode (linear application) of applying in a stripe form, a mode (point group application) of applying in dot form may be appropriately used for the application mode. Among them, the point group application is most preferable since the tightening force can be appropriately dispersed. The application mode may have patterns of different types coexisting or patterns of different sizes coexisting.

The knitted material having stretchability is suitably used for the fabric of the main part. The knitted material includes lateral knitted material and circular knitted material, and may be either as long as a satisfactory stretchability is obtained.

The stretchability of the first tightening part is lower than that of the main part, as described above.
Specifically, the contraction force at the time of 30% extension of the first tightening part is preferably greater than or equal to two times and smaller than or equal to ten times the contraction force at the time of 30% extension of the main part. More preferably, it is greater than or equal to three times and smaller than or equal to eight times.

In the present invention, the contraction force is measured with the following method. Assume that a direction of highest stretchability is an A direction and a direction perpendicular to the A direction is a B direction. A rectangular sample having a length of 16 centimeters parallel to the A direction and a width of 2.5 centimeters in the direction (B direction) perpendicular to the A direction is collected for the main part and the first tightening part. With respect to the sample, both ends on the short side of the rectangle were clamped at a clamping interval of 10 centimeters and a 0 to 80% stretching process was repeated for three times with the tension test equipment, and the recovery force at the time point of 30% extension at the time of third unloading, that is, the contraction force, was measured based on the cut strip method of JIS L 1018.

If the tightening part is formed by overlapping the patch of (1) on the main part, the sample of the tightening part is in a state where the material for forming the main part and the patch are overlapped, and is not a single patch.

The contraction force of the first tightening part is preferably greater than or equal to two times and smaller than or equal to ten times the contraction force of the main part in both A direction and B direction measured in the relevant method. This is because the stimulation to apply on the muscle serving as the tightening part may lack if smaller than two times, and the stimulation on the muscle becomes too strong and may give an uncomfortable feeling if greater than ten times.

An area of each first tightening part arranged on the left and the right is preferably 10 cm² to 300 cm². More preferably, it is 30 cm² to 100 cm². The contacting pressure that stimulates the left and right shoulder deltoid muscles may be small if smaller than 10cm². The shoulder may feel an oppressing feeling if greater than 300 cm².

The first tightening part is preferably arranged one each on the left and the right, but may be in plurals separately each on the left and the right.

The shape of each first tightening part on the left and the right is a shape in which an upper part is branched to a shoulder region side and a sleeve region side, and left and right side edges at a lower part are converged towards a middle of the lower end when divided to the shoulder region spreading to the back side and the sleeve region spreading to the arm side with the shoulder in between.

In the case of a shape in which the upper part shape is branched to the shoulder region and the sleeve region, the movement of the arms is not suppressed and the load by the first tightening part can be acted without being dispersed by continuing the lower parts.

The back side of the sleeve is sewed to the back body section as a raglan sleeve, and the first tightening part is preferably arranged at the raglan sleeve.

As described above, since the first tightening part is arranged covering the shoulder and the sleeve at the back, if the sewing line of the back body section and the sleeve is the set-in line along the shoulder hole, the first tightening part is positioned over the sewing line, which is not preferable as sewing becomes difficult and it is not visually satisfactory. Furthermore, in the case of the raglan sleeve with the back body section and the back sleeve sewed at the raglan line, the stretchability is suppressed at the sewing line, and the action of the first tightening part positioned on the raglan sleeve side can be further enhanced.

The sleeve may be a short sleeve or a long sleeve, and is a sleeve in which at least the first tightening part can be arranged.

The front side of the sleeve may be a sleeve to be sewed to the front body section along the shoulder hole, that is, the set-in line. In this case, the outer appearance on the front side has a shape similar to a normal shirt and can be made elegant.

The front part may be a raglan sleeve, similar to the back part.

The sleeve portion may be a long sleeve, and a second tightening part of band-shape for alleviating arm fatigue, which passes obliquely through a common digital extensor muscle from an inner side of a wrist towards the outer side of an elbow, may be arranged on a forearm from the wrist to the elbow. The second tightening part has a lower stretchability than the main part.

The second tightening part may be arranged in the same mode as the first tightening part, or the tightening part may be arranged in other modes described in (1) to (4).

The contraction force of the second tightening part may be the same as the first tightening part or may be different, but the stretchability is lower than the sleeve main part.

In recent years, the personal computer and the like are often operated continuously for a long period time, in which case fatigue accumulates at the portion from the wrist continuing to the fingers to the elbow and the arm may feel heavy, which tends to lower an operating efficiency. Similarly, when performing tasks that use arms such as with a hasher or a component assembly worker, the fatigue easily accumulates at the arm thereby lowering the operating efficiency.

If the second tightening part of band shape for alleviating arm fatigue that passes obliquely through the common digital extensor muscle from an inner side of a wrist towards the outer side of an elbow is arranged at the forearm from the wrist to the elbow, the common digital extensor muscle can be stimulated, and the holding force of the forearm can be enhanced thereby aiding the movement of the arm. As a result, the fatigue of the arm can be reduced and the lowering of the operating efficiency can be suppressed.

The second tightening parts respectively have a width of 3 cm to 10 cm. More preferably, it is in the range of 4 cm to 5 cm. The effect of reducing the fatigue may be low if smaller than 3cm, and a constriction feeling may arise if greater than 10 cm.

The garment is an upper garment, and is preferably an undershirt, or an outer upper garment including those for sports and rehabilitation. Furthermore, it is also suitably used in a swimming wear, a leotard, and the like.

The garment of the present invention is not limited to the upper garment on the top side, and the front and back body sections may continuously include a lower body section reaching to at least the femoral area, or may couple the lower body sections in attachable/detachable manner through a connecting portion.

The main part of the lower body section is preferably made from a stretch material.

The lower body section preferably includes a band-shaped third tightening part along a waist, a fourth tightening part for holding down a middle of a buttocks arranged up to a position corresponding to a sacral bone in continuation to a middle of the back of the third tightening part, and a band shaped fifth tightening part arranged along a hip joint at a base of the femoral area on the back side.

The third to fifth tightening parts may have lower stretchability and higher contraction force in a mode similar to the first tightening part, or may have lower stretchability in other modes described in (1) to (4).

The contraction force of the third to fifth tightening parts may be the same as the first tightening part or may be different, but the stretchability is lower than the main part.

As described above, the lower body section may be arranged integrally with the upper body section by continuing the main parts, or a connection piece may be coupled. In this case, it may be opened and closed at a crotch portion.

The upper body section and the lower body section may be coupled in attaching/detaching manner with a button, a fastener, and the like.

If the lower body section is arranged, and the fourth tightening part is arranged at the site corresponding to the sacral bone of the lower body section, that is, at the position immediately before the gluteal cleft, the position of the sacral bone is positioned at the middle of the buttocks and thus in contact with the middle of the buttocks stronger than other sites to securely hold it down, thereby stabilizing the center position of the lower body.

Normally when fatigue accumulates, the upper body tends to lean forward and the lower body tends to lean backward. In this case, the third tightening part holds down the middle of the buttocks from the back side, and thus the buttocks is suppressed from being on the back side and leaning forward in the standing posture. As a result, a straight posture is realized, the center of gravity position becomes higher and the waist position becomes higher. In particular, the standing posture and the walking posture are aided to a straight posture with a synergetic effect with the action of straightening the back muscle on the top side.

With the arrangement of the band shaped third tightening part along the waist, the body can be securely held at the center position in the up and down direction. A boundary position of the lower body and the upper body can be securely held with the third tightening part, the lower body can be induced to a vertical state with the force of pushing the sacral bone with the lower body section, the chest is elevated with the first tightening part and the back muscle is straightened, so that the standing posture becomes straight and a preferable appearance can be presented.

Furthermore, the fifth tightening part arranged along the hip joint of the back extends from both sides of the crotch portion towards a lower abdomen at the back side of the base of the femoral area to support the hip joint. The fifth tightening part enables a balanced walking without causing unbalance in the body at the time of walking, and pushes the femoral area forward at the time of waking thereby aiding the movement of the leg during walking and making the step larger.

On the upper body side, the swing of the left and right hands to the back side becomes larger with the first tightening part, and furthermore, the movement of the leg at the time of walking becomes large, so that walking lively in a correct walking posture can be realized.

A sixth tightening part continuing to the third tightening part passing obliquely upward towards at a middle of the front part along the hip joint on the front side in continuation to the fifth tightening part and then passing obliquely upward towards the back side, and a seventh tightening part arranged in continuation to the third tightening part to the lower abdomen of the middle of the front sandwiched by the front end edges of the sixth tightening parts on the left and right sides and having the lower end position up to the intermediate position of a length reaching a crotch portion from the waist line are preferably arranged at the lower abdomen at the front part of the lower body section.

Similar to the first to fifth tightening parts, the sixth and seventh tightening parts also have lower stretchability than the main part, and are formed in one of the modes of (1) to (4).

If the sixth tightening part is arranged along the hip joint on the front side in continuation to the fifth tightening part along the hip joint at the base of the femoral area on the back side, the movement of the femoral area can be supported at the time of walking.

If the tightening part is arranged on the lower side than the base of the femoral area at the front part, a force that suppresses the movement of the leg to the front side at the time of walking tends to occur, but the load is not applied on the femoral area in the present invention since the hip joint at the back side of the femoral area that becomes the supporting point of the movement of the leg to the front side is securely held down but the entire femoral area is not held down. Furthermore, since the fourth and fifth tightening parts of band-shape are arranged along the hip joint at the base of the femoral area from the back side to the lower abdomen side, the walking can be aided without causing unbalance in time of walking.

If the portion that is in contact with the lower abdomen on the front side entirely has a high tightening force with the sixth tightening part and the seventh tightening part, the force can be easily applied to the lower abdomen and a stable state can be reliably realized with the front and the back of the lower body. Thus, a secure and clear posture is obtained in commuting and outing without feeling tired.

The lower end position of the seventh tightening part is a position immediately before reaching the local area that bulges out for male garments so as not to generate tightening feeling and uncomfortable feeling.

The second invention provides a garment of top and bottom set with a top garment including an upper body section and a bottom garment including a lower body section.

The top garment corresponds to the upper body section of the first invention and the bottom garment corresponds to the lower body section of the first invention, which are arranged as separate bodies and can be worn as a set.

In other words, the top garment includes a main part which includes front and back body sections and sleeves extending from the front and back body sections towards both arm sides over shoulders integrally or as separate bodies, and a pair of first tightening parts separately placed in left and right areas located at the positions of right and left deltoid muscles covering a boundary of the wearer's shoulder and upper arm in the back side. The front and back body sections and the upper arm sections of the sleeves are made from at least two kinds of stretch materials, which differ in stretchability from each other, including a material for the main part and a material for the first tightening parts having a lower stretchability than the main part.

The bottom garment includes a lower body section reaching to at least a femoral area; and a main part of the lower body section is made from a stretch material. The lower body section includes a fourth tightening part for holding down a middle of a buttocks arranged at a position corresponding to a sacral bone, and a fifth tightening part arranged in a band shape along a hip joint at a base of the femoral area on the back side. The fourth tightening part and the fifth tightening part have lower stretchability than the main part.

When the top garment and the bottom garment are worn as a set, the fatigue on both the upper body side and the lower body side can be reduced, and a physically and mentally cleared state can be achieved.

Since the chest is elevated with the top garment, the back muscle is straightened, and the middle of the buttocks is held down and the hip joint is held with the bottom garment, the standing posture becomes a straight and correct posture, and furthermore, the step becomes greater at the time of walking and the hands can be swung to the back side greatly, thereby aiding the correct walking posture. In addition, the step and the walking speed can also be increased.

### [Effect of the Invention]

According to the garment of the first invention, the first tightening parts are arranged on the left and the right separately at the site of the left and right shoulder deltoid muscles, thereby stimulating the shoulder deltoid muscles of the wearer so as to be conscious, inducing the left and right scapulae to expand to the back side without generating a tightening feeling on the back side to straighten the back. As a result, an increase in heart rate and breath rate can be suppressed or reduced, and the body function can be enhanced in simply wearing. The step and the walking speed can also be increased.

According to the set garment of the second invention, the center of gravity of the lower body can be stabilized with the bottom garment, and hence the walking posture can be induced to a straight and correct posture together with the top garment. Furthermore, the femoral area can be pushed forward and the walking is aided, so that the step increases at the time of walking and the swing of the hands to the back side can be made large with the top garment, whereby the walking posture can be aided to the correct posture with a large step and a large swing of the hands.

### BRIEF DESCRIPTION OF THE DRAWINGS

Fig. 1 shows an undershirt of a first embodiment of the present invention, where (A) is a rear view and (B) is a front view.
Fig. 2 is an enlarged view of main parts of Fig. 1(B).
Fig. 3 shows a variant of the first embodiment, where
   (A) is an enlarged view of main parts of a first variant,
   (B) is an enlarged view of main parts of the first variant,
   (C) is a rear view of a third variant, and (D) is a rear view of a seventh variant.

Fig. 4 shows a top-bottom connected garment of a second embodiment, where (A) is a rear view and (B) is a front view.
Fig. 5 shows a variant of the second embodiment, where
   (A) is an enlarged view of main parts of a first variant, and (B) is an enlarged view of main parts of a second variant.
Fig. 6 shows a third embodiment, where (A) is a back view and (B) is a front view.
Fig. 7 is a view showing a marker position given to a subject in an experimental example.
Fig. 8 is a photo showing a walking posture of the subject in the experimental example.
Fig. 9(A) is a photo showing a posture when the garment of the present invention is worn, and Fig. 9(B) is a photo showing a posture when not worn.
Figs. 10(A) to 10(C) are views describing a three-dimensional operating analysis of the experimental example.
Figs. 11 (A) to 11(C) are graphs showing a change in center of gravity during walking of the experimental example.
Fig. 12 is a graph showing a change in a breath rate during walking.
Fig. 13 is a graph showing average data of a change in a breath rate of the subject.
Fig. 14 is a graph of a heart rate of the experimental example.
Fig. 15 is a graph showing the change by elapse of time of an average heart rate and an exercise intensity of all subjects.
Figs. 16(A) to 16(F) are graphs showing a heart rate of six subjects.
Fig. 17 is a graph showing a heart rate and a fluctuation of the heart rate for one subject.
Fig. 18 is a view showing a conventional example.
Fig. 19 is a view showing another conventional example.

### BEST MODE FOR CARRYING OUT THE INVENTION

One example of an embodiment of the present invention will be described below with reference to the drawings.

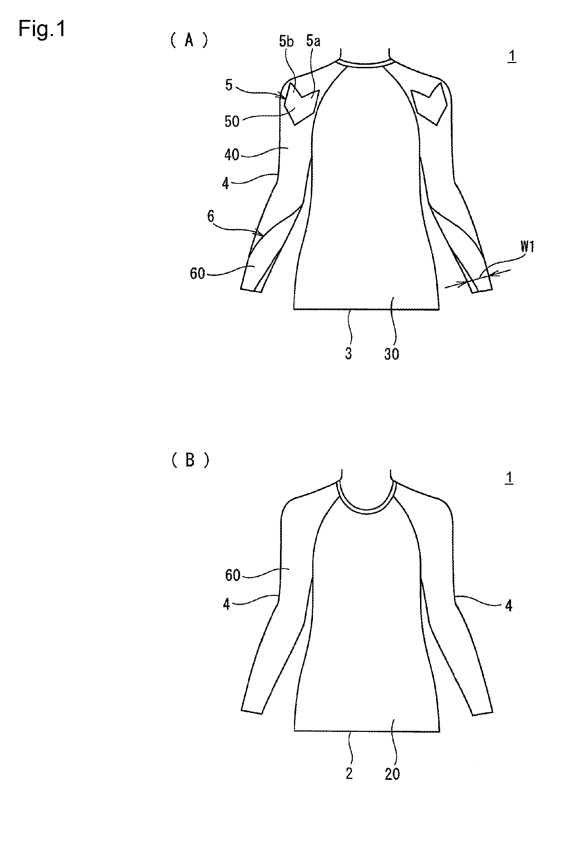

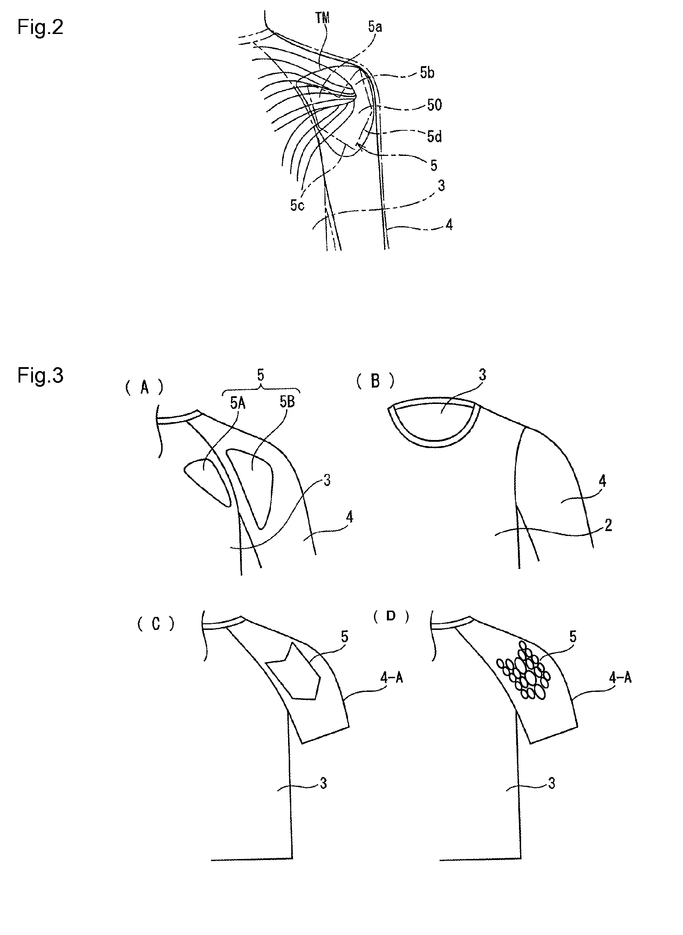

Fig. 1 and Fig. 2 show an undershirt according to a first embodiment.

As shown in Figs. 1A and 1B, the first embodiment relates to a long-sleeved undershirt 1. The undershirt 1 includes a front body section 2, a back body section 3, and left and right long sleeves 4. The front and back body sections 2, 3 have a length that reaches to at least the waist line, and have a length that reaches to the stomach in the present embodiment. Main parts 20, 30, 40 of the front and back body sections 2, 3 and the sleeves 4 are made from a knit knitted fabric of first stretch material. The left and right sleeves 4 are respectively formed from one cloth, where the lower end edge is sewed to obtain a tubular shape.
The sleeves 4 are raglan sleeves, where the shoulder side end edges of the front and back body sections 2, 3 and the shoulder side end edges of the sleeves 4 are sewed to a raglan line.

As shown in a rear view of Fig. 1A and Fig. 2, first tightening parts 5 are arranged at positions corresponding to left and right shoulder deltoid muscles TM at the outer surface of the upper section of the rear (back) of the sleeve 4 serving as the raglan sleeve. The pair of left and right first tightening parts 5 separately arranged at the shoulder deltoid muscles on both side, are made from a second stretch material having lower stretchability than the main parts 20, 30 and 40. The first tightening part 5 is formed by overlapping a patch 50 made from the second stretch material on the outer surface of the main part 40 of the sleeve 4, and attaching the same by sewing the peripheral edge of the patch 50 to the main part 40.

Thus, the undershirt 1 is made from two types of stretch materials, the first stretch material with satisfactory stretchability for forming the main parts 20 to 40, and the second stretch material having lower stretchability for forming the first tightening part 5. The first tightening parts placed at the positions of left and right shoulder deltoid muscles are in contact with the shoulder deltoid muscles of the wearer stronger than other areas thereby stimulating the shoulder deltoid muscles.

The left and right first tightening parts 5 are each positioned to bridge between a shoulder region and a sleeve region with the shoulder in between on the back surface side of the raglan sleeve. The shape of each first tightening part 5 is a substantially heart shape where the upper part includes a branched portion 5a branched to the shoulder region side and a branched portion 5b branched to the sleeve region side, and the left and right side edges 5c, 5d at the lower part converge towards the middle of the lower end.

The area of each first tightening part is in the range of 10 cm² to 300 cm², and is about 30 cm² in the present embodiment.

The contraction force of the main part and the first tightening part is defined by being measured through the measurement method described above. In other words, assuming a direction of highest stretchability as A direction and a direction perpendicular to the A direction as B direction, a rectangular sample having a length of 16 centimeters parallel to the A direction and a width of 2.5 centimeters in the direction (B direction) perpendicular to the A direction is collected for the main part and the first tightening part. With respect to the sample, both ends on the short side of the rectangle sample were clamped at a clamping interval of 10 centimeters and a 0 to 80% stretching process was repeated for three times with the tension test equipment, and the recovery force at the time point of 30% extension at the time of third unloading was calculated as "UP30" based on the cut strip method of JIS L 1018.

In the present embodiment, the fabric of the main parts 20 to 40 is made from a circular knitted fabric, the A direction of highest stretchability being a horizontal direction, and the fabric of the first tightening part 5 is made from a warp knitted fabric, the A direction of highest stretchability being a vertical direction.

An UP30 in the A direction of the main parts 20 to 40 is 50 cN and the UP30 in the A direction of the first tightening part 5 is 145 cN. The B direction of the main parts 20 to 40 is the vertical direction, the B direction of the first tightening part 5 is the horizontal direction, the UP30 in the B direction of the main parts 20 to 40 is 55 cN, and the UP30 in the B direction of the first tightening part 5 is 151 cN. In other words, the first tightening part has a contraction force of three times the main part and gives a lower stretchability in both directions of the vertical direction and the horizontal direction.

The contraction force of the first tightening part is not limited to three times the main part, and may be made stronger to eight times or about ten times.

The main parts 20, 30 and 40 have a thin thickness. Specifically, the fabric weight is 100 to 200 g/m².

At the left and right sleeves 4, a band-shaped second tightening part 6 for alleviating the arm fatigue that passes obliquely through the common digital extensor muscle from the inner side of the side end of the wrist to the outer side of the elbow is provided at the forearm from the wrist to the elbow. The second tightening part 6 is formed by a cloth 60 made from the same second stretch material as the first tightening part 5, the cloth 60 being overlapped on the outer surface of the main part 40 and the peripheral edge thereof is sewed. The width W1 of the second tightening part 6 is in the range of 3 cm to 10 cm, and is 4.5 mm width in the present embodiment. The second tightening part 6 may not be arranged even if the sleeves 4 are long sleeves.

In the undershirt 1 having the above configuration, the main parts 20, 30 and 40 can be entirely fitted to the body of the wearer since they are made from the thin first stretch material that has a satisfactory stretch, and hence a tightening feeling does not occur and an uncomfortable feeling does not occur when worn.

The wearer can always be conscious of the shoulder deltoid muscles since the first tightening parts 5 strongly are in contact with the shoulder deltoid muscles at the back side, and can have the left and right scapulae adducted to move closer to each other and induce the left and right shoulders to spread wide towards the back side so that the wearer does not take the posture in which the left and right scapulae are inclined forward at the time of fatigue and stress. Furthermore, even if the left and right scapulae incline forward to a slouched posture, the left and right first tightening parts 5 do not follow the forward inclination of the left and right scapulae, and the force in a direction of pulling the left and right scapulae to the back side act thereby enlarging the rib cage elevate and inducing the back muscle in a straightening direction. Regarding the positions of the left and right hands also, the upper arm and the elbow can be induced to the back side to be positioned along the side of the body, so that the upper body can be held in a state where the rib cage is elevated and the back muscle is straightened.

As a result, an increase in heart rate and the breath rate can be suppressed and even reduced, as shown in the examples to be described later. The back muscle can be straightened and the head can be held at the vertical position along the backbone so as not to take the posture leaned forward as a whole but to inductively take a straight posture.

Furthermore, since the second tightening part 6 is arranged to pass the common digital extensor muscle obliquely at the sleeve 4, the common digital extensor muscle can be stimulated and the holding force of the forearm section can be enhanced. As a result, the fatigue of the arm can be reduced and the lowering of the operating efficiency can be suppressed.

Fig. 3A shows a first variant of the first embodiment. In the first variant, each of the left and right first tightening part 5 provided at each area of the left and right shoulder deltoid muscle is separated to a back side part 5-A and a shoulder side part 5-B with a space. Thus, effects similar to the first embodiment can be obtained even if the first tightening part 5 is formed by two configuring elements as long as the shoulder deltoid muscle can be stimulated.

Fig. 3B shows a second variant of the first embodiment. In the second variant, a sewing line of the front body section 2 and the sleeve 4 is a set-in line along an armhole, and the sewing line of the back body section 3 and the sleeve 4 is the raglan line same as the above. In other words, the outer appearance on the front side is the shape of a typical undershirt as a typical set-in line since the first tightening part is not attached to the front part.

Fig. 3C shows a third variant of the first embodiment. In the third variant, only the first tightening part 5 is arranged and the second tightening part 6 is not arranged since the sleeve 4-A is short sleeve.

Furthermore, in the first embodiment, the first tightening part has the patch 50 provided by being sewed to the outer surface of the main part 40, but may be attached to the inner surface side of the main part.

In a fourth variant of the first embodiment, the stretch material for the patch of the first and second tightening parts is the same material as the stretch material of the main part. The patch of the first and second tightening parts is applied with resin adhesive at the peripheral edge of the patch and applied in dot form at the interior region to securely attach the first and second tightening parts to the main part.

The hot melt resin of polyurethane, polyester, polyolefin, styrene, and the like is used for the resin adhesive.

Thus, the stretchability becomes lower and the contraction force becomes stronger than the main part even if the patches of the first and second tightening parts are the same as the fabric of the main part by overlapping and interposing the resin adhesive.

In a fifth variant of the fifth embodiment, an elastic resin is applied to the relevant portion of the main part 40 of the sleeve to provide the first and second tightening parts instead of using the patch for the first and second tightening parts 5, 6 formed with the patch shown in Fig. 1.

A cross-linking agent or a penetrating agent may be contained in the main agent including silicone, urethane, acryl, or nylon for the elastic resin, and applied all over the portion to become the first and second tightening parts by transfer.

The application method is not limited to transfer, and may be spraying, screen printing, rotary printing, and the like. The application mode may be a linear application to a stripe shape, pointed application to dot shape, and the like.

Therefore, the first and second tightening parts do not stand out on the outer appearance by arranging the first and second tightening parts without attaching the patch. Furthermore, the feel against the skin is more satisfactory than that in a case where the first and second tightening parts are arranged by attaching the patch on the inner surface side.

Similar to the fifth variant, a sixth variant of the first embodiment also does not form the first and second tightening parts with the patch but form the tightening parts by changing the fabric. The knitting manner of reducing the loop length or increasing the number of sinker loops in the unit area to suppress the deformation of the loop is adopted in the first and second tightening parts.

Fig. 3D shows a seventh variant of the first embodiment. In the seventh embodiment, the first tightening part in the third variant of Fig. 3C is formed by applying the elastic resin to a point group form. The tightening force can be appropriately dispersed by applying in a point group form.

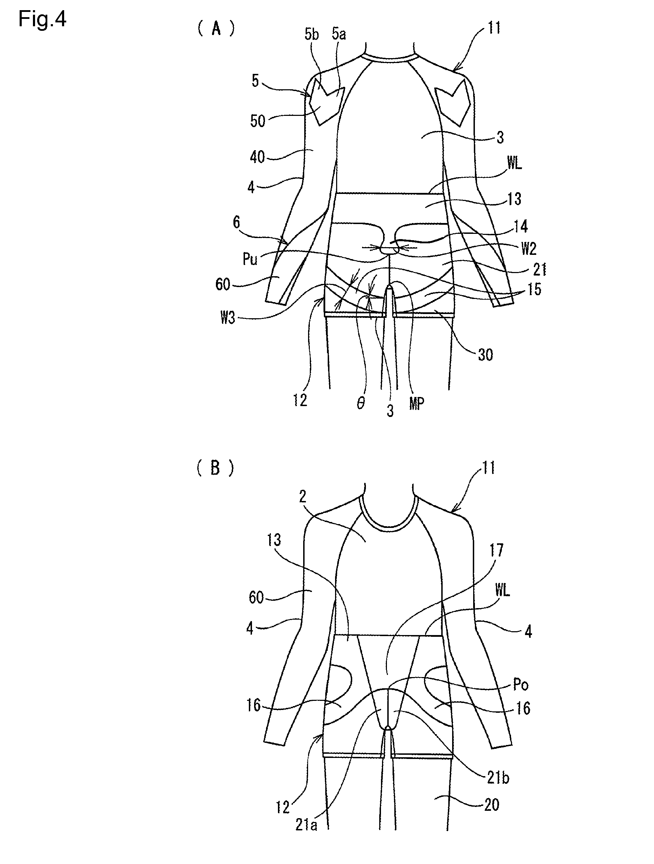

Figs. 4A and 4B show a garment according to a second embodiment.

In the second embodiment, a top-bottom connected garment 11 is formed by continuing a bottom 12 that covers the lower body section from the waist line to the femoral area of the upper leg to the top 11 that covers the upper half of the body having the same shape as the undershirt 1 of the first embodiment. The top-bottom connected garment 11 is a garment for men but may be for women.

A main part 121 of the bottom 12 of the lower body section is formed by sewing three patterns of left and right main parts 21a and a front central part 21b, made from the first stretch material to the center on the back part and both sides on the front part. The lower end of the main part 21 is positioned at substantially the middle in the up and down direction of the femoral area.

The bottom 12 is provided with a band-shaped third tightening part 13 having a width of about 5 cm from an upper end of the waist line WL over the entire periphery. The third tightening part 13 has a lower stretchability than the main part, and hence strongly fits around the waist even if a rubber band and the like is not arranged along the waist line WL. The rubber band may be attached along the waist line WL.

At the back (buttocks) side, a fourth tightening part 14 for holding the middle of the buttocks is arranged up to the position corresponding to the sacrum, that is, the position reaching to the gluteal cleft in continuation to the middle on the back of the third tightening part 13, and a fifth tightening part 15 of band-shape inclined to the upper side is arranged along the hip joint at the base of the femoral area.

A sixth tightening part 16 continuing to the fifth tightening part 15 and passed obliquely upward towards the lower abdomen at the middle of the front part along the hip joint on the front side and then passed obliquely upward towards the back side and continued to the third tightening part 13 is arranged at the front part of the bottom 12, and a seventh tightening part 17 is arranged at the lower abdomen of the middle of the front part sandwiched by the front end edges of the left and right sixth tightening parts 16.

The sixth and seventh tightening parts 16, 17 are continued to the third tightening part 13 around the waist.

The lower end position Po of the seventh tightening part 17 at the middle of the lower abdomen is the intermediate position of the length from the waist line to the crotch portion and is terminated at a position immediately before reaching the local area that bulges out.

The cutting and the sewing are carried out so as to bulge out towards the front side while dividing the front central part 21b to the left and the right by the position of reaching the tip of the crotch portion from the seventh tightening part 17.

The third to the seventh tightening parts 13 to 17 are formed by a patch made from the second stretch material with low stretchability same as the first and second tightening parts 5, 6 of the first embodiment, and are sewed to the outer surface of the main part 21.

The lower end position Pu of the fourth tightening part 14 for holding down the middle of the buttocks is in the range of 8 cm to 16 cm to the lower side from the waist line WL, and is positioned at 11 cm in the present embodiment. Furthermore, the maximum width W2 in the left and right direction is 4 cm to 10 cm, and is 6 cm in the present embodiment.

The left and right fifth tightening arts 15 extending in a band shape along the hip joint at the base of the femoral area are inclined to the lower abdomen side from the position of 5 cm to 10 cm (7 cm in the present embodiment) to the lower side from the tip of the crotch portion MP with an upward angle θ at 10 degrees to 60 degrees (40 degrees in the present embodiment). The upward angle θ is an angle with respect to the perpendicular line of the sewing line with the crotch portion, as shown in the figure.

The width W of the fifth tightening part 15 is 4 cm to 10 cm, and is 6 cm in the present embodiment.

The sixth tightening part 16 arranged on the left and the right of the lower abdomen in continuation to the front end of the fifth tightening part 15 is passed obliquely upward towards the lower abdomen at the middle of the front part along the hip joint at the base of the femoral area on the front part side, and has the lower end edge continued to the lower end edge of the seventh tightening part 17.

The upper side edge is passed obliquely upward with the upper end of the upward inclined part directed to the back side, and continued to the third tightening part 13.

Thus, the third to the seventh tightening parts 13 to 17 are arranged at the bottom 12 and the patch of low stretchability is not attached from the left and right hips towards the left and right back bulge-out parts. Therefore, the buttocks line bulges out thereby realizing a satisfactory appearance without inhibiting the movement of a lower back and without applying a load on the left and right bulge-out parts.

At the bottom 12 having the above configuration, the fourth tightening part 14 is positioned at the middle of the buttocks at the site corresponding to the sacral bone and hence in contact with the middle of the buttocks more strongly than other sites and acts to securely hold it down, whereby the center of gravity position of the lower body can be stabilized.

The body is securely held at the center position in the up and down direction of the body by the band-shaped third tightening part 13 along the waist part, and the lower body is induced to the vertical state with the force of pushing the sacral bone by the fourth tightening part 14 at the bottom so that the center of gravity position becomes higher and the waist line can be held high.

Furthermore, in addition to elevating the chest and straightening the back muscle with the first tightening part 1, the standing posture becomes straight thereby presenting a preferred outer appearance.

Since the sixth and seventh tightening parts 16, 17 are arranged at the lower abdomen, the lower abdomen can be securely held down, and the center of gravity position of the body is supported from the front and the back to stabilize the body trunk with the holding down action by the fourth tightening part 14 positioned at the middle on the back side.

As the fourth tightening part 14 is not extended at the position along the gluteal cleft, the gluteal cleft of the cleavage of the buttocks can be appeared as a definite line thus not inhibiting the visual look.

When walking, the fifth and sixth tightening parts 15, 16 are arranged along the hip joint at the base of the femoral area for support, thereby aiding the walk without causing unbalance in the body with the fifth and sixth tightening parts 15, 16 as the supporting point during the walk.

Since the femoral area is pushed forward by the existence of the fifth tightening part 15 on the back side, the step becomes greater at the time of walking. Furthermore, the movement of the leg at the time of walking becomes greater with the swing of the left and right hands to the back side becoming greater by the first tightening part 5 on the top side, which contribute to health enhancement by walking.

The first to seventh tightening parts of the second embodiment are formed by sewing the patch, but may be formed by applying an elastic resin as described in the fifth variant of the first embodiment, or may be formed by changing the fabric as regulated in the sixth variant.

In the top and bottom connected garment of the second embodiment, the stretchability of the first stretch material of the main part may be made lower and the stretchability of the second stretch material of the first to seventh tightening parts may be further made lower than the undershirt of the first embodiment to realize a leotard that can be suitably worn during walking and during exercise.

The lower end of the bottom 12 may be extended to below the knee.

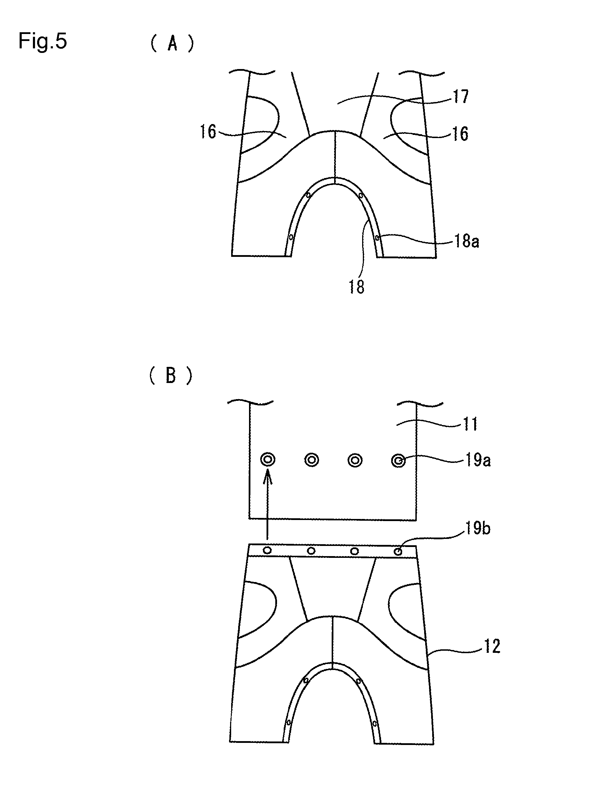

Figs. 5A and 5B show a variant of the second embodiment.

In the first variant shown in Fig. 5A, an open/close part 18 is arranged at the crotch portion and is opened/closed by attaching a hook 18a thereby facilitating urination and bowl movement.

In the second variant shown in Fig. 5B, the top 11 and the bottom 12 are separated, and are coupled in attachable/detachable manner with a button 19a and a button hole 19b arranged at the lower side of the top 11 and the upper end of the bottom 12.

Therefore, if the top 11 and the button 12 can be arbitrarily coupled and separated, the wearer can wear only either one thereby enhancing the convenience.

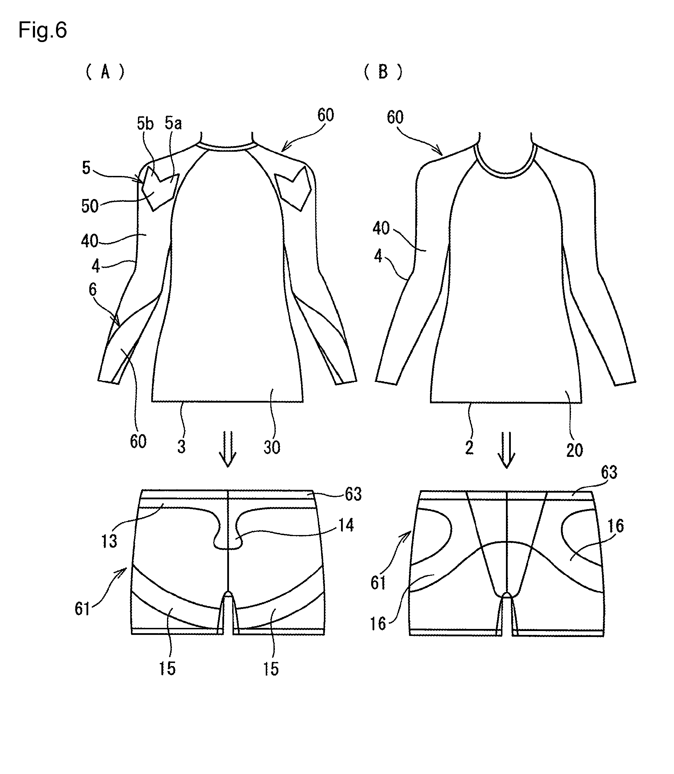

Fig. 6 shows a garment of a third embodiment.

The third embodiment is similar to a separating type shown in Fig. 5B, but coupling means is not arranged at the top side and the bottom side.

A top garment 60 has a configuration similar to the top 11, and a bottom garment 61 has a configuration similar to the bottom 12, but the third tightening part 13 is separated from the top side and a rubber band 63 is accommodated at a position along the waist line WL at the upper end.

The description on other configurations and effects will be omitted by denoting the same reference numerals as the second embodiment.

### [Experimental example]

The heart rate, the breath rate, and the motion capture of the subject were measured for when the top garment 60 and the bottom garment 61 (hereinafter abbreviated as garment in the present invention) of the third embodiment were worn by the subject and for when not worn.

In the measurement of the breath rate and the motion capture, the subjects were three males in the ages of thirties, forties, and fifties who do not have abnormality in the movement function. The temperature at the time of measurement was 25°C and the humidity was 60%.

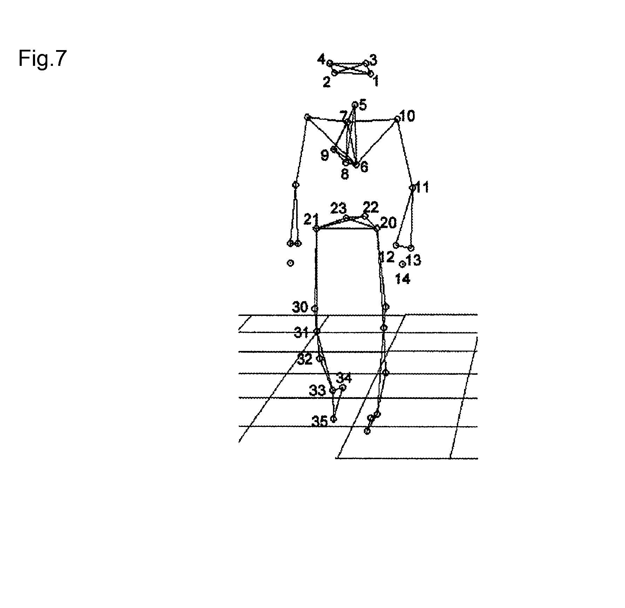

In the measurement of the breath rate, the ventilation amount was measured using an exhaled air gas analyzer manufactured from Inter-Reha Co. Ltd. In the measurement of the motion capture, the MX-F optical three-dimensional operating analysis system manufactured by VICON was used, and a marker was given to each position 1 to 35 shown in Fig. 7 to the subject and the garment of the present invention worn by the subject. The positions 1 to 35 of the marker are as follows.
1: left front head, 2: right front head, 3: left back head, 4: right back head, 5: seventh spinous process of cervical vertebra, 6: tenth spinous process of dorsal vertebra, 7: center point of left and right sternoclavicular joint, 8: ensiform cartilage, 9: right back part, 10: acromioclavicular joint, 11: left lateral epicondyle of humerus, 12: front part of left wrist, 13: back part of left wrist, 14: left fingertip, 15: right acromioclavicular joint, 16: right lateral epicondyle of humerus, 17: front part of right wrist, 18: back part of right wrist, 19: right finger tip, 20: left anterior superior iliac spine (ASIS), 21: right ASIS, 22: left posterior superior iliac spine (PSIS), 23: right PSIS, 24: left femoral area, 25: left femoral lateral epocondyle, 26: left leg region, 27: left ankle, 28: left foot heel, 29: left second metatarsal bone head, 30: right femoral area, 31: right femoral lateral epocondyle, 32: right leg region, 33: right ankle, 34: right foot heel, 35: right second metatarsal bone head.

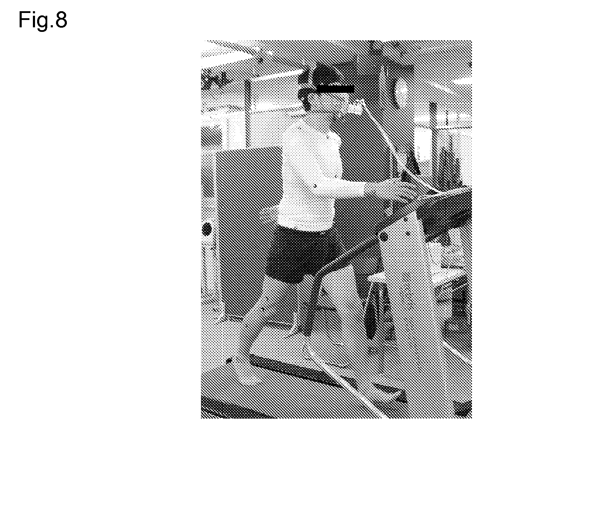

Three types of measurements were then carried out during the exercise in the program. After three minutes has elapsed in a resting state while sitting (hereinafter referred to as sitting and resting posture), a resting state while standing (hereinafter referred to as standing and resting posture) was maintained for five minutes. Thereafter, as shown in the photo in Fig. 8, the walking experiment was conducted on an automatic walking machine (EDM-T tread mill system manufactured by Zebris). The walking experiment was continued for three minutes respectively at the speed of 1 km, 3 km, and 7 km per hour. Finally, the standing and resting posture was continued for five minutes.

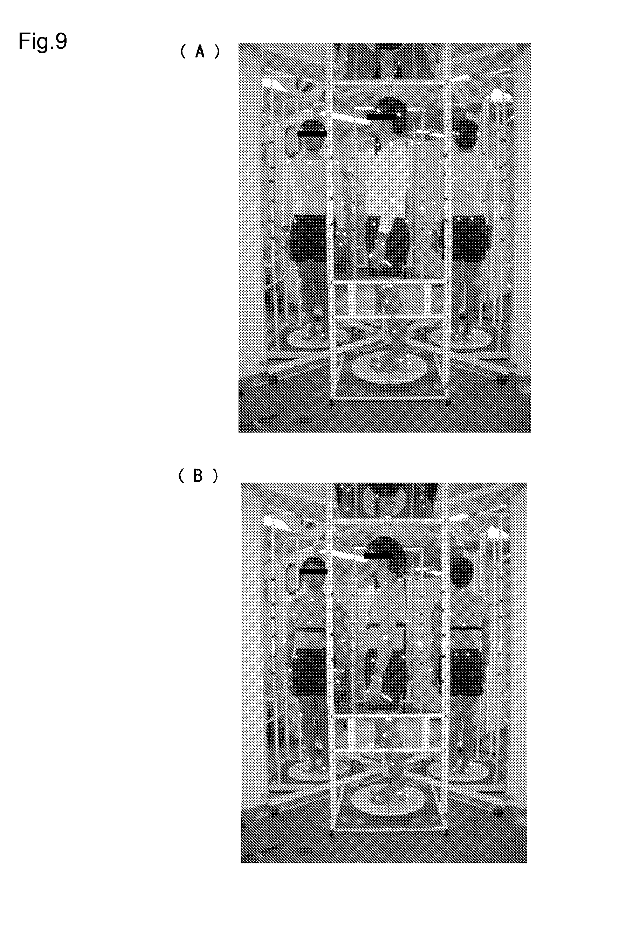

The last standing and resting posture after walking in the experiment is shown in Fig. 9. Fig. 9A shows a standing posture when wearing the top garment 60 and the bottom garment 61 of the third embodiment, and Fig. 9B shows a standing and resting posture when not wearing the garments.

When wearing the garment of the present invention of Fig. 9A, the left and right scapulae adduct in the direction of approaching each other so that the left and right shoulders are induced to the back side, thereby elevating the rib cage. The head is held on a vertical line of the spine, so that the body trunk is extended.

When the garments are not worn as shown in Fig. 9B, the posture is rather slouched, the rib cage is narrowed, the head is not held on the vertical line of the spine, and the body trunk is not extended as opposed to when the garments are worn as shown in Fig. 9A.

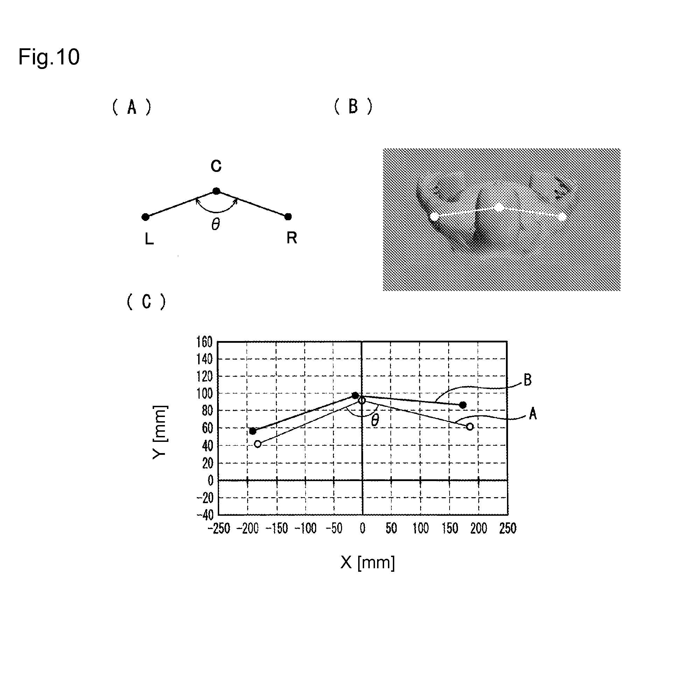

Figs. 10A, 10B and 10C show the three-dimensional operating analysis data of VICON. The three points in Figs. 10A, 10B, 10C indicate R: right shoulder, C: center point of left and right sternoclavicular joint, L: left shoulder from the right side.

As shown in Figs. 10A and 10B, an angle θ of both shoulders from the center point of the left and right sternoclavicular joint is calculated from the coordinate positions of the three points in the XY coordinate plane.

The horizontal axis of the graph in Fig. 10C is the average position [mm] in the X axis (left and right) direction from the center of the lower back, and the vertical axis is the average position [mm] in the Y axis (front and back) direction from the center of the pelvis. A line A shows the angle θ on the back surface side when the garments of the present invention are worn, and a line B when not worn, showing that when the angle becomes narrow, the rib cage is elevated and the left and right scapulae are adducted.

As shown in Fig. 10C, the angle of the line A when the garments of the present invention are worn is narrower than the angle θ of line B of when not worn.

Therefore, it was found that if the top garment including the undershirt of the first embodiment of the garment of the present invention is worn, the rib cage elevates, the back muscle becomes straight, and the body trunk extends straightly.

Furthermore, the change in the center of gravity position at the time of walking was calculated in the three-dimensional operating analysis data of VICON.

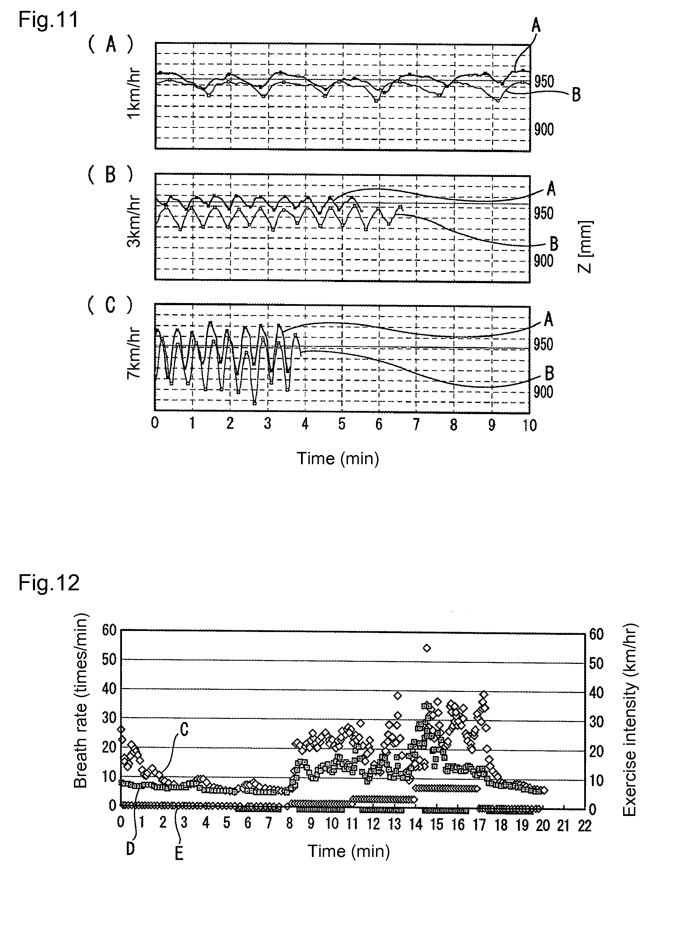

Figs. 11A, 11B and 11C show the data of the temporal change in the center of gravity position for the walking cycle of three to four rounds of the belt of the automatic walking device at the time of walking at 1 km, 3 km and 7 km per hour. The vertical axis of the graph is the position [mm] of the center of gravity (Z axis) from the floor surface. The line A is for when the garments of the present invention are worn, and the like B is for when not worn.

It was found that the center of gravity position is higher when worn, as indicated by the line A, than when not worn at the time of walking. Furthermore, the fluctuation in the up and down positions is relatively small in the line A than in the line B, and the variation in the up and down movement tends to be reduced at the time of walking. Therefore, it was found that walking can be carried out without causing unbalance in the body.

Fig. 12 shows the data in which the change in the breath rate due to change in the walking speed was measured with the exhaled gas when the garments of the present invention were worn.

The sitting and resting posture for three minutes or longer, the standing and resting posture for three minutes, walking for three minutes at 1 km per hour, walking for three minutes at 3 km per hour, walking for three minutes at 7 km per hour, and lastly standing and resting posture for three minutes were conducted in order, and the data during this time was measured.

In Fig. 12, the elapsed time (minutes) is shown on the horizontal axis, the breath rate (times/minute) is shown on the vertical axis on the left side, and the exercise intensity (km/hr) is shown on the vertical axis on the right side, and the result for one subject is shown.

In Fig. 12, a line C shows the change in breath rate when not worn, a line D shows the change in breath rate when the garments of the present invention are worn, and a line E shows the intensity of walking exercise.

The average value of the measured breath rate between 30 seconds to two minutes after the start of the exercise is shown.

The ventilation amount measured using the exhaled gas analyzer for when worn and for when not worn shown in Fig. 12 is shown in the following table 1.

[Table 1]

**Table 1**

| | breath rate when not worn (times/min) | breath rate when worn (times/min) |
|---|---|---|
| Pre | 6.8 ± 0.7 | 4.8 ± 0.1 |
| 1km/hr | 22.4 ± 1.5 | 13.3 ± 1.6 |
| 3km/hr | 20.7 ± 4.4 | 12.7 ± 1.9 |
| 7km/hr | 29.5 ± 4.4 | 19.3 ± 5.8 |
| Post | 9.2 ± 1.5 | 7.5 ± 0.6 |

Here, "Pre" means at the time of the standing and resting posture for five minutes before the walking test on the tread mill, and "Post" means at the time of the standing and resting posture after the walking test on the tread mill.

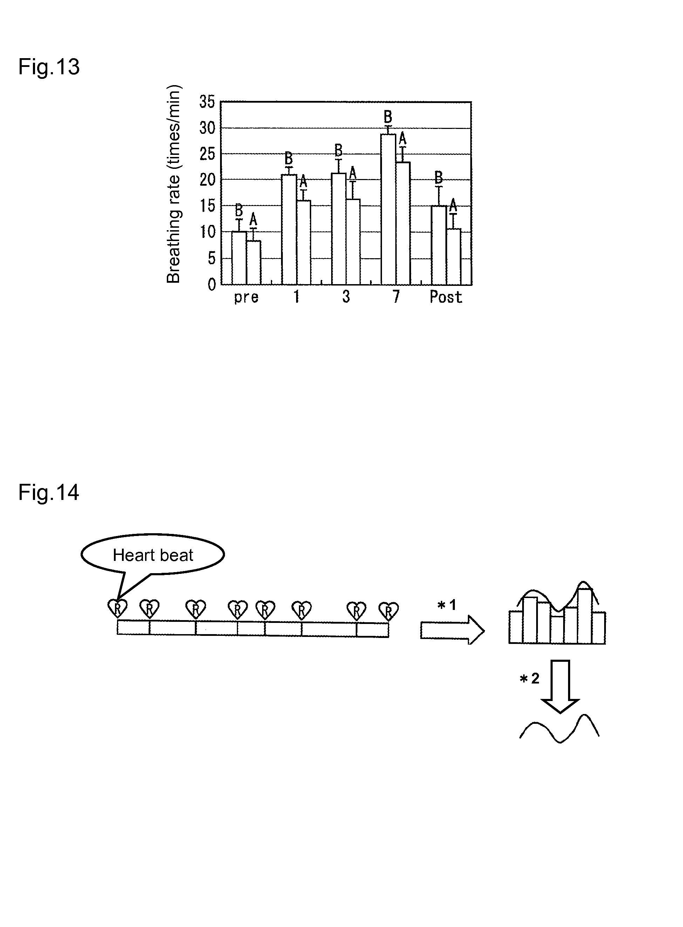

Fig. 13 shows the average data for the change in breath rate for three subjects. A is for when worn, and B is for when not worn. The results of Fig. 13 are tabulated, as shown in table 2.

**Table 2**

| | breath rate when not | breath rate when |
|---|---|---|
| | worn (times/min) | worn (times/min) |
| pre | 10.2 ± 2.3 | 8.3 ± 2.3 |
| 1km/hr | 21.2 ± 1.5 | 16.1 ± 1.9 |
| 3km/hr | 21.0 ± 2.7 | 16.2 ± 3.5 |
| 7km/hr | 28.7 ± 1.6 | 23.5 ± 2.8 |
| Post | 15.2 ± 4.9 | 19.7 ± 2.9 |

As shown in Figs. 12, 13 and the tables 1, 2, the breath rate at the time of walking was reduced when the garments of the present invention were worn compared to when not worn. From such results, it was found that the rib cage elevates, the back muscle straightens and the body trunk straightly extends by wearing the top garment constituted of the undershirt of the first embodiment.

In the measurement of the heart rate, six healthy males (between 31 to 51 years old, average age of 39 years old) were the subject.

The heart rate meter RS800sd manufactured by Polar Co. was used to measure the heart rate.

The subjects walked on the tread mill with the heart rate meter attached. The temperature at the time of measurement was 25° and the humidity was 60%.

Similar to the measurement of the breath rate, in the measurement of the heart rate, after elapse of three minutes in the sitting and resting posture, the standing and resting posture was maintained for five minutes and the walking experiment was conducted on the automatic walking machine (EDM-T tread mill system manufactured by Zebris Co.). The walking experiment was continued for three minutes respectively at the speed of 1 km, 3 km and 7 km per hour. Lastly, the standing and resting posture was continued for five minutes.

The measured hear rates were sampled one minute at a time, and the fluctuation in the heart rate was analyzed using the Memcale of GMS Co.

The heart rate fluctuation was analyzed in the following manner. As shown in Fig. 14, the heart rates were stacked for every time interval (RR interval) of the heart rate for every one beat (*1 process of Fig. 14), the stacked bar graph was converted to a waveform data (*2 process of Fig. 14), and the waveform was broken into sinusoidal waves. R in Fig. 14 is the time point the heart rate appeared. A specific component in the frequency of the sinusoidal wave was given attention, where 0.04 to 0.15 Hz was set as HF, HF being the index of the parasympathetic nerve and the LF (LF/HF) being the index of the sympathetic nerve.

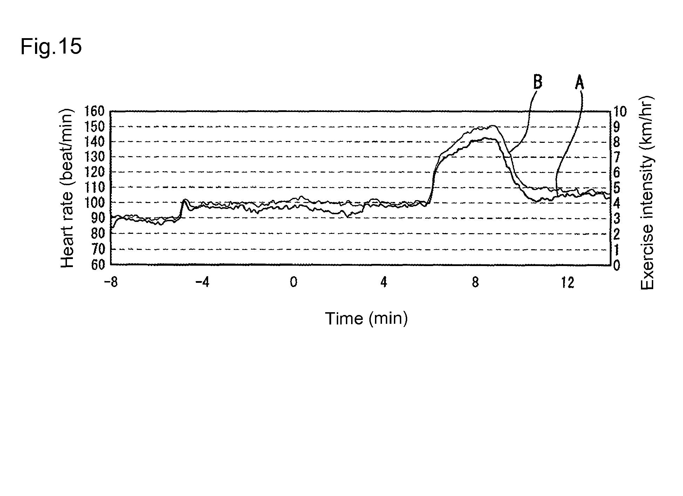

Fig. 15 shows the change by elapse of time in the average heart rate and the exercise intensity for all the subjects. The horizontal axis shows the elapsed time (minutes), the vertical axis on the left side shows the heart rate (beat/minute), and the vertical axis on the right side shows the exercise intensity (km/hr). The line A is for when worn, and the line B is for when not worn.

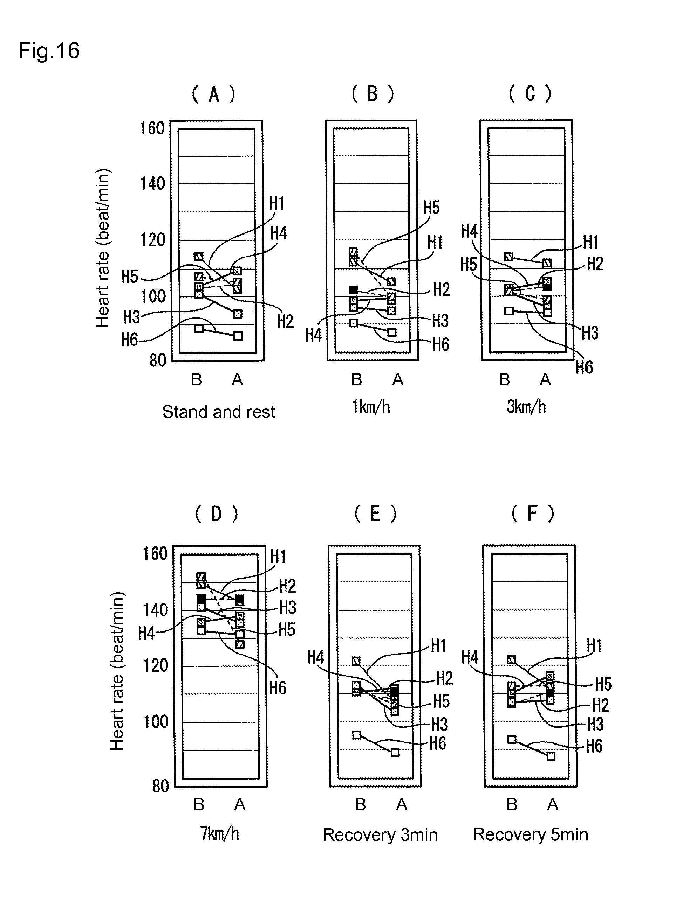

Figs. 16A to 16F show the heart rate for subjects 1 to 6. Each graph is a graph for (A) standing and resting posture before exercise, (B) walking at 1 km per hour, (C) walking at 3 km per hour, (D) walking at 7 km per hour, (E) standing and resting posture at the time point of elapse of three minutes after exercise, and (F) standing and resting posture at the time point of elapse of five minutes after exercise. The vertical axis of each graph is the heart rate (beat/min). The lines H1 to H6 are each data of the subjects 1 to 6. The right side in each graph is the data when the garment of the present invention is worn, and the left side is the data when the garment of the present invention is not worn.

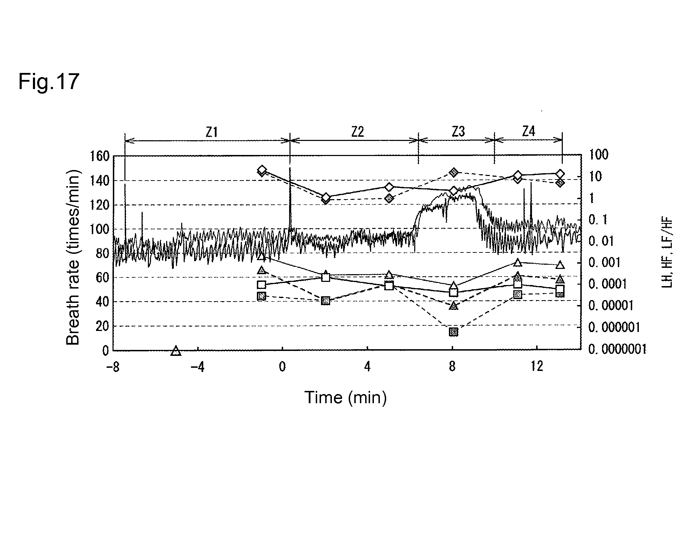

Fig. 17 shows the detailed data of the subject 1. Similar detailed data were also collected for the subjects 2 to 6, and the average data of Fig. 15 was obtained based on each data.

In Fig. 17, the line connecting the triangular points indicates LF, the line connecting the square points indicates HF, and the line connecting the diamond points indicates LF/HF. The activities of the sympathetic nerve and the parasympathetic nerve were analyzed from the lines of LF, HF, and LF/HF.

As shown in the heart rate data of Figs. 15 to 17, the heart rate was reduced when the garment of the present invention was worn compared to when not worn in the resting posture of the region Z1 at the time of the sitting and resting posture, and the standing and resting posture.

In the region Z2 until elapse of six minutes from the start of walking, there were barely difference from the region Z1 at the time of standing and resting posture, and the heart rate was reduced when the garment of the present invention was worn compared to when not worn.

An apparent difference was found in the region Z3 from six minutes to ten minutes after the start of walking for when worn and for when not worn, and the heart rate was reduced when worn.

There were barely variation in the heart rate in the region Z4 at the time of standing and resting posture after walking, and the heart rate was reduced when the garment of the present invention was worn compared to when not worn.

Therefore, it was found that the heart rate tends to reduce at all times including at the time of sitting and resting posture, at the time of the standing and resting posture, at the time of walking at 1 km, 3 km and 7 km per hour, and at the time of recovery of the standing and resting posture after walking.

From the data LF, HF and LF/HF showing the fluctuation in the heart rate shown in Fig. 17, it was found that a difference in activity clearly appears in the region Z3 for when the garment of the present invention was worn and when not worn, where the sympathetic nerve activity increased and the parasympathetic nerve activity continued when worn.

Some subjects whose heart rate greatly reduced in walking at 7 km per hour suggested the possibility that walking can be carried out in a form closer to the resting state where the parasympathetic nerve activity does not reduce.

Some subjects suggested that the RR interval width of the heart beat increased and that deep breathing was performed when the garment of the present invention was worn.

The step and the walking speed were measured using the operating analyzer.
- measurement device: "MAC3D System" manufactured by Motion Analysis
- subject: five healthy male in twenties to fifties
- experimental method
   A walking path of eight meters on a carpet was walked back and forth, and data was acquired three minutes after the start of walking so that the steady walking state can be measured avoiding the influence at the beginning of walking.
- analyzing method
   The elevated degree of the chest, which is important with regards to the posture, and the movement distance of the heel, which is important with regards to the walking action, were analyzed among those the data for the action of 35 areas such as the joint of the whole body were acquired.

### • experimental result

The movement distance and the movement speed for one period of the periodic walking action can be measured by focusing on the action of one heel. Focusing on the action of one heel, the heel lands on the floor, kicks the floor, moves away from the floor, and moves to the next landing area. The movement distance from the landing to the next landing is substantially twice the "one step in daily language", that is, "step" in the "movement distance for one period of walking". The time from "landing" to the next "landing" is the "time for one period of walking", and thus the "speed of walking" can be calculated.

Spread in the step was seen in substantially all the subjects by wearing the wear of the present invention. The spread was 11 cm at a maximum. The walking speed also substantially increased.

[Table 3]

**Table 3 step**

| | average increase of step (cm) | average increasing rate of step(%) |
|---|---|---|
| only shirt is worn | 2.97 | 4.51 |
| only pants is worn | 1.27 | 1.89 |
| top and bottom are worn | 6.57 | 10.68 |

**Table 4 walking speed**

| | average increase of speed(km/h) | average increasing rate of speed(%) |
|---|---|---|
| only shirt is worn | 0.27 | 6.24 |
| only pants is worn | 0.03 | 1.98 |
| top and bottom are worn | 0.33 | 8.60 |

From the experimental data above, when the garment of the present invention is worn, the breath rate, the heart rate and the fluctuation in the heart rate can be reduced, and the step and the walking speed can be increased. This effect is recognized when the breath rate and the heart rate are reduced by arranging the first tightening part and inducing so as to elevate the rib cage. Furthermore, if the rib cage is elevated, the back muscle straightens, the center of gravity moves upward when walking, the legs can reliably move from the hip joint, and the step and the walking speed can be increased. The garment of the present invention can be suitably used as the underwear that is worn daily where the top side is the undershirt and the bottom side is the pants, but it is not limited to the underwear, and may be an outer. For instance, it is suitably used as an exercise outer worn during walking or during other exercises, a rehabilitation outer worn during gait training and the like.

### Description of Symbols

- 1: undershirt
- 2: front body section
- 3: back body section
- 4: sleeve
- 5: first tightening part
- 6: second tightening part
- 11: top
- 12: bottom
- 13: third tightening part

- 14: fourth tightening part
- 15: fifth tightening part
- 16: sixth tightening part
- 17: seventh tightening part
- 20, 30, 40: main part made of first stretch material
- 50: patch
- 60: top garment
- 61: bottom garment
- 63: rubber band

## Claims

1. A garment comprising:
a main part which includes front and back body sections, and sleeves extending from the front and back body sections towards both arm sides over shoulders; and
a pair of first tightening parts separately placed in left and right areas located at the positions of right and left deltoid muscles covering a boundary of the wearer's shoulder and upper arm in the back side, wherein
the front and back body sections and the upper arm sections of the sleeves are made from at least two kinds of stretch materials, which differ in stretchability from each other, including a material for the main part and a material for the first tightening parts having a lower stretchability than the main part.

2. The garment according to claim 1, wherein the first tightening part is formed from one of the means selected from (1) to (4):
(1) a patch made from the same material as or a different material from the main part is overlapped on an outer surface or an inner surface of the main part, and then sewed, or adhered or thermal compression bonded using a resin adhesive;
(2) a patch made from a different material from the main part is filled and arranged in an opening provided in the main part, and joining end edges are sewed;
(3) a fabric is changed from the main part with the same material as or a different material from the main part; and
(4) an elastic resin is applied to the main body.

3. The garment according to claim 1, wherein the first tightening part is a point group, the point group being formed with means (5) or (6):
(5) a fabric is changed from the main part with the same material as or a different material from the main part; and
(6) an elastic resin is applied to the main body.

4. The garment according to any one of claims 1 to 3, wherein a contraction force at the time of 30% extension of the first tightening part is greater than or equal to two times and smaller than or equal to ten times the contraction force at the time of 30% extension of the main body.

5. The garment according to any one of claims 1 to 4, wherein an area of each first tightening part arranged on the left and the right is 10 cm² to 300 cm², the first tightening parts being provided one each on the left and the right, or in plurals separately each on the left and the right.

6. The garment according to any one of claims 1 to 5, wherein a shape of each first tightening part on the left and the right arranged between a shoulder region and a sleeve region with the shoulder in between is a shape in which an upper part is branched to a shoulder region side and a sleeve region side, and left and right side edges at a lower part are converged towards a middle of the lower end.

7. The garment according to any one of claims 1 to 6, wherein the back side of the sleeve is sewed to the back body section as a raglan sleeve, the first tightening part being arranged on the raglan sleeve.

8. The garment according to any one of claims 1 to 7, wherein the sleeve is a short sleeve or a long sleeve, and is a sleeve in which a front part of the sleeve is sewed to the front body section along a shoulder line or a raglan sleeve.

9. The garment according to any one of claims 1 to 8, wherein the sleeves are long sleeves, and a second tightening part of band-shape for alleviating arm fatigue, which pass obliquely through a common digital extensor muscle from an inner side of a wrist towards the outer side of an elbow, is arranged on a forearm from the wrist to the elbow.

10. The garment according to claim 9, wherein the second tightening parts respectively have a width of 3 cm to 10 cm.

11. The garment according to any one of claims 1 to 10, being one of an undershirt, an outer upper garment including those for sports and rehabilitation, a swimming wear, and a leotard.

12. The garment according to any one of claims 1 to 10, wherein
the front and back body section continuously includes a lower body section reaching to at least a femoral area or couples to the lower body section in attachable/detachable manner through a connecting portion;
a main part of the lower body section is made from a stretch material; and
the lower body section includes a band-shaped third tightening part along a waist, a fourth tightening part for holding down a middle of a buttocks arranged up to a position corresponding to a sacral bone in continuation to a middle of the back of the third tightening part, and a band shaped fifth tightening part arranged along a hip joint at a base of the femoral area on the back side.

13. The garment according to claim 12, wherein a sixth tightening part continuing to the third tightening part passing obliquely upward towards a lower abdomen at a middle of the front part along the hip joint on the front side in continuation to the fifth tightening part and then passing obliquely upward towards the back side, and a seventh tightening part arranged in continuation to the third tightening part at the lower abdomen of the middle of the front part sandwiched by the front end edges of the sixth tightening parts on the left and right sides and having the lower end position up to the intermediate position of a length reaching a crotch portion from a waist line are arranged at a front part of the lower body section.

14. The garment according to claim 13, wherein the lower end position of the seventh tightening part is a position immediately before reaching a local area that bulges out for male garments.

15. A garment comprising a garment of top and bottom set with a top garment and a bottom garment; wherein
the top garment includes a main part which includes front and back body sections and sleeves extending from the front and back body sections towards both arm sides over shoulders, and a pair of first tightening parts separately placed in left and right areas located at the positions of right and left deltoid muscles covering a boundary of the wearer's shoulder and upper arm in the back side,
the front and back body sections and the upper arm sections of the sleeves are made from at least two kinds of stretch materials, which differ in stretchability from each other, including a material for the main part and a material for the first tightening parts having a lower stretchability than the main part;
the bottom garment includes a lower body section reaching to at least a femoral area;
a main part of the lower body section is made from a stretch material;
the lower body section includes a fourth tightening part for holding down a middle of a buttocks arranged at a position corresponding to a sacral bone, and a fifth tightening part arranged in a band shape along a hip joint at a base of the femoral area on the back side; and
the fourth tightening part and the fifth tightening part have lower stretchability than the main part of the lower body section.
